# EUROPÄISCHE PATENTANMELDUNG

(11) **EP 4 132 045 A1**
(43) Veröffentlichungstag der Anmeldung: **08.02.2023**
(21) Anmeldenummer: 21189122.1
(22) Anmeldetag: 02.08.2021
(51) Int. Cl.: H04W 12/03, H04W 12/50, A61M 5/24, A61M 5/178, H04W 84/18, H04B 5/00

(54) **INJEKTIONSSYSTEM MIT VEREINFACHTER DATENÜBERTRAGUNG**

(71) Anmelder: Ypsomed AG, 3401 Burgdorf (CH)
(72) Erfinder: Urbanek, Leos, 3012 Bern (CH); Maechler, Silas, 3270 Aarberg (CH); Mangold, Stefan, 3097 Liebefeld (CH)
(74) Vertreter: Meier Obertüfer, Jürg

(57) **Zusammenfassung**

Die Erfindung betrifft ein Injektionssystem in welchem Daten von einem Verabreichungsgerät für Medikamente über eine drahtlose Bluetooth-Verbindung zu einem Mobilgerät und von dort über eine Netzwerkverbindung an einen Server-Computer übertragen werden. Ein neuartiges Verfahren erlaubt es die Verabreichungsdaten auf dem Verabreichungsgerät zu verschlüsseln und über den Bluetooth Broadcast-Kanal zu versenden ohne ein Pairing durchzuführen. Die Verabreichungsdaten werden erst auf dem Server-Computer entschlüsselt und dem Verabreichungsgerät zugeordnet. Dieser Prozess kann dadurch unterstützt werden, dass sowohl das Mobilgerät, als auch das Verabreichungsgerät den Bluetooth Broadcast-Kanal beobachten und eine Abschätzung vornimmt wie weit der jeweilige Sender der übertragenen Daten vom Mobilgerät oder Verabreichungsgerät entfernt ist.

## Beschreibung

### TECHNISCHES GEBIET

Die vorliegende Erfindung betrifft das Gebiet der medizinischen Injektionssysteme, insbesondere der smarten Injektionssysteme, welche Daten von einem Verabreichungsgerät für Medikamente an andere Geräte übertragen können.

### HINTERGRUND DER ERFINDUNG

Immer mehr Krankheiten und Leiden werden behandelbar. Es finden immer mehr teure Medikamente und Behandlungen den Weg in die Therapie. Deshalb ist eine Kostenkontrolle bei gleichzeitiger Sicherstellung von Patientensicherheit und Datenschutz immer wichtiger. Einerseits wird versucht Heimtherapien auch bei aufwendigeren Therapien zu ermöglichen, was Gesundkosten reduzieren kann. Andererseits sollte insbesondere bei teureren Therapien sichergestellt werden können, dass die Medikamente auch bei Heimtherapie durch die Patentinnen und Patienten korrekt verwendet werden, und weiter die Therapietreue der Patientinnen und Patienten gesichert ist.

Aus dem Stand der Technik sind Injektionssysteme bekannt, bei welchen Verabreichungsgeräte Informationen zur Verwendung der Verabreichungsgeräte via Bluetooth an ein Mobilgerät senden und das Mobilgerät die Informationen zumindest zu einem Teil weiter an einen oder mehrere entfernte Server sendet. Da diese Informationen sensible Patienteninformationen umfassen können, müssen diese Informationen gesichert übertragen werden, um sie vor fremdem Zugriff zu schützen. So muss die Verbindung zwischen Mobilgerät und Verabreichungsgerät geschützt sein, wie auch die Verbindung zwischen Mobilgerät und entfernten Servercomputern. Hinzu kommt, dass die Information auch auf den jeweiligen Geräten und Computern immer sicher aufbewahrt werden müssen.

Bei der Verbindung zwischen Mobilgerät und Verabreichungsgerät kommt typischerweise eine verschlüsselte Bluetooth-Verbindung zum Einsatz. Dazu müssen Mobilgerät und Verabreichungsgerät einen Kopplungsprozess durchlaufen, welcher unter anderem eine eindeutige Identifikation und Zuordnung zwischen den Kommunikationspartnern ermöglicht. Im Bluetooth-Kommunikationsprotokoll wird diese Kopplung "Pairing" genannt. Um die Verbindung weiter abzusichern, kann auf Applikationsebene zwischen Mobilgerät und Verabreichungsgerät eine weitere Verschlüsselung etabliert werden. Diese mehrstufige Absicherung erfordert häufig einen durch eine benutzende Person angestossenen Kopplungs-, Pairing- und Schlüsseltauschprozess. Für einfachere Injektionsgeräte wie Autoinjektoren kann dadurch die Absicherung der Verbindung zwischen Injektionsgerät und Mobilgerät mehr Zeit in Anspruch nehmen als die eigentliche Injektion. Für Patientinnen und Patienten ist eine mehrstufige Absicherung der Verbindung auch mit dem Risiko von Fehlmanipulationen verbunden, insbesondere bei Vorliegen einer Sehschwäche. Es besteht also ein Bedürfnis nach einer einfach zu bedienenden und trotzdem sicheren Informationsübertragung bei Injektionsgeräten, die zum Beispiel nur für eine Injektion verwendet werden und im Anschluss an die Benutzung entsorgt werden.

Der Begriff "Produkt", "Medikament" oder "medizinische Substanz" umfasst im vorliegenden Zusammenhang jede fliessfähige oder fluide medizinische Formulierung, welche geeignet ist zur kontrollierten Verabreichung mittels einer Kanüle oder Hohlnadel in subkutanes oder intramuskuläres Gewebe, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder co-formulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP 1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

Unter den Begriffen "Injektionssystem", Injektionsgerät oder "Injektor" wird in der vorliegenden Beschreibung eine Vorrichtung verstanden, bei der die Injektionsnadel nach erfolgter Abgabe einer kontrollierten Menge der medizinischen Substanz aus dem Gewebe entfernt wird. Somit verbleibt bei einem Injektionssystem oder bei einem Injektor im Unterschied zu einem Infusionssystem die Injektionsnadel nicht über einen längeren Zeitraum von mehreren Stunden im Gewebe. Ein Injektionssystem kann neben dem eigentlichen Injektionsgerät eine oder mehrere weitere Injektionsgeräte umfassen. Bluetooth ist als Funktechnologie hinlänglich bekannt. Etwas weniger bekannt sind sogenannte Bluetooth Beacons, zu Deutsch Baken oder Leuchtfeuer, wobei der Einfachheit halber im Folgenden der englische Begriff gebraucht wird. Mit Beacon wird ein Sender oder Empfänger bezeichnet, der auf der Bluetooth Low Energy BLE oder auch Bluetooth Smart Technology BST basiert. Im Grunde genommen ist dies eine Funktechnologie, die als Weiterentwicklung von Bluetooth verstanden werden kann. Wie bei jeder Funktechnik braucht man zunächst mindestens einen Sender und mindestens einen Empfänger. Die Sender werden hier als "Beacons" bezeichnet. Ihre Reichweite beträgt rund zehn Meter, je nach Entwicklungsstand und Hersteller. Bei der Datenübertragung zwischen den Geräten mit BLE wird weit weniger Energie aufgewandt als bei älteren Varianten von Bluetooth, was beispielsweise einen geringeren Batterieverbrauch beim Sender zur Folge hat. Auch die Kosten des Transfers bleiben niedrig. Große Datenmengen eignen sich nicht für den Austausch, weil die Übertragungsrate relativ gering ist. Die Beacons selbst werden per Batterie oder seltener direktem Stromanschluss betrieben. Es sind bereits vielerorts Beacons im Einsatz, um Besucher in einem Laden zu lokalisieren und ortsabhängige Angebote auf das jeweilige Endgerät zu schicken. So finden sich zum Beispiel Beacons im Hamburger Flughafen im Einsatz, die die verbrachte Zeit in verschiedenen Geschäften messen und mit Prämien belohnen.

Die Bluetooth Beacons können insbesondere als Sender für kleine Datenmengen genutzt werden. Dabei werden die Daten nicht in einer Punkt zu Punkt-Verbindung übertragen, sondern der Bluetooth Beacon sendet die Daten im Broadcast-Modus, als sogenanntes Advertising-Paket, welches von allen empfangenden Bluetooth-Geräten, die sich in Funkreichweite des sendenden Beacons befinden, empfangen werden. Das bedeutet, empfangende Geräte müssen nicht via Bluetooth-Pairing mit dem Beacon vor der Datenübertragung gekoppelt und/oder auf irgendeine Weise identifiziert werden. In frühen Varianten von Bluetooth kann ein Advertising-Paket beispielsweise 31 Bytes an Daten enthalten. Dabei können die Daten zum Beispiel eine Sender-Identifikation und weitere Daten umfassen. Der Begriff "Empfang" umfasst in diesem Dokument nicht zwingend die Interpretation der Daten. So können Datenpakete mit verschlüsseltem Inhalt empfangen und weitergeleitet werden, ohne den Inhalt zu erkennen oder zu interpretieren. In der Telekommunikation ist dem Fachmann auch seit jeher wohlbekannt, dass aus dem Empfang von Signalen unabhängig vom Inhalt der Kommunikation gewisse Information gewonnen werden kann, die zur Steuerung der Übertragung nützlich ist. Das einfachste Beispiel dazu ist die Messung der Signalstärke beim Empfang drahtloser Signale, welche in vielen Kommunikationssystemen zur Regelung der Antennenverstärkung oder der Sendeleistung benutzt wird.

Für Details zu Bluetooth im Allgemeinen, Bluetooth Low Energy und Beacons, welche Bluetooth als Technologie verwendet, wird auf die Bluetooth-Spezifikation verwiesen, welche unter https://www.bluetooth.com/specifications/specs abgerufen werden kann.

### DARSTELLUNG DER ERFINDUNG

Es ist eine Aufgabe der Erfindung, alternative Injektionssysteme bereitzustellen, welche eine vereinfachte und sichere Übertragung von Injektionsdaten von einem Injektionsgerät auf einen entfernten Server erlauben.

Die Aufgabe wird gelöst durch ein Verfahren und ein System gemäss den unabhängigen Ansprüchen. Weiterentwickelte, vorteilhafte Lösungen der Aufgabe sind den abhängigen Ansprüchen, sowie der Beschreibung und den Figuren zu entnehmen.

In einer Ausgestaltung der Erfindung umfasst die Erfindung ein System zur sicheren Übertragung von medizinischer Information. Die Information wird dabei von einem Verabreichungsgerät zuerst an ein Mobilgerät und dann vom Mobilgerät weiter an einen entfernten Server oder Computer übertragen.

Das Verabreichungsgerät kann dabei insbesondere ein Patch-Injektor, ein Autoinjektor, ein Injektions-Pen oder eine Infusionspumpe sein. Weitere mögliche Verabreichungsgeräte sind Geräte zum Inhalieren von Substanzen über die Atemwege, sowohl für medizinische Zwecke oder als Genussmittel. Ebenfalls können Verabreichungsgeräte erfindungsgemäss modular aus voneinander lösbaren oder unlösbaren Komponenten aufgebaut sein. Typische Module sind dabei eine Reservoir-Einheit mit dem Reservoir zum Einweggebrauch, ein Kommunikationsmodul, sowie eine wiederverwendbare Antriebseinheit mit dem Antrieb. Beispielhaft werden folgend für die Beschreibung der Erfindung ein Patch-Injektor und ein Autoinjektor genommen.

Ein solcher Autoinjektor umfasst eine Vorrichtung zum Verabreichen von fluidem Medikament, wie sie dem Fachmann von den marktüblichen Autoinjektoren bekannt sind. Weiter umfasst der Autoinjektor eine elektronische Steuerung. Die Steuerung kann mit ebenfalls im Autoinjektor vorsehbaren Sensoren verbunden sein, und deren Messsignale entgegennehmen und verarbeiten. Weiter ist die Steuerung mit einem im Autoinjektor vorgesehen Speicher verbunden, in welchem die Steuerung Daten speichern kann. Im oder am Autoinjektor ist eine Bluetooth-Einheit angeordnet, welche mit der Steuerung verbunden ist, und über welche Daten drahtlos gesendet und empfangen werden können. Insbesondere kann die Bluetooth-Einheit Daten in einem Broadcast-Modus in Form von oben beschriebenen Advertising-Paketen senden und empfangen. In einer Ausgestaltung funktioniert die Bluetooth-Einheit exklusiv als Beacon im Broadcast-Modus. In einem so ausgestatteten Autoinjektor kann über die Sensoren eine Medikamentenverabreichung überwacht werden. Die Steuerung kann die Sensorsignale entgegen nehmen, speichern und/oder via die Bluetooth-Einheit drahtlos verbreiten.

Erfindungsgemäss umfasst das System ein Mobilgerät. Dabei kann es sich um ein Smart Phone, einen PDA, ein Tablet, ein Notebook oder ein ähnlich geeignetes Gerät handeln. Das Mobilgerät enthält erfindungsgemäss auch eine Bluetooth-Einheit, sowie eine Netzwerkeinheit zur Kommunikation mit entfernten Geräten. Dabei kann es sich um eine Ethernet-Einheit, also drahtgebundene Netzwerkeinheit, eine drahtlose Netzwerkeinheit für ein WLAN oder ein ähnliches System, eine Mobilfunkeinheit oder auch um eine Mobiltelefonie-Einheit beliebiger Generation handeln. Beides, Bluetooth wie auch Netzwerk, sind bei den erwähnten Geräteklassen übliche Funktionalitäten, weit verbreitet und dem Fachmann wohlbekannt.

Erfindungsgemäss umfasst das System mindestens einen weiteren entfernten Computer oder Server. Insbesondere muss der mindestens eine Computer nicht unbedingt ein realer Computer sein, sondern es können auch eine oder mehrere Cloud-Instanzen, also virtuelle Computer oder Container, gemeint sein. Der mindestens eine Computer oder Server Computer umfasst mindestens einen, allenfalls virtuellen, Prozessor, einen Speicher und eine Anbindung an ein Netzwerk. Diese Komponenten sind bei virtuellen und realen Servern übliche Komponenten und bedürfen keiner weiteren Ausführung zum Verständnis der Erfindung.

Der Autoinjektor, als ein nicht einschränkendes Beispiel für ein erfindungsgemässes Injektions-oder Infusionsgerät, hat wie erwähnt einen Speicher, in welchem die Steuerung Daten ablegen kann. Weiter ist in diesem Speicher zumindest ein kryptografischer Schlüssel abgelegt, mit welchem die Steuerung bei Bedarf Daten verschlüsseln oder zertifizieren kann und sie entweder verschlüsselt im Speicher ablegen kann oder verschlüsselt über die Bluetooth-Einheit des Autoinjektors verbreiten kann. Im Speicher ist sodann auch eine Kennung des Autoinjektors abgelegt. In einem modular aufgebauten Verabreichungsgerät kann der Speicher auch mehrteilig und auf die verschiedenen Module verteilt angeordnet sein. Insbesondere kann der Schlüssel in einem Speicher in einer Reservoir-Einheit abgelegt sein, um die Zuordnung zum Medikament zu gewährleisten.

Nun kann die Steuerung des Autoinjektors Verabreichungsdaten, welche zum Beispiel mittels der Sensoren aufgezeichnet und gespeichert wurden, verschlüsseln und zumindest zusammen mit der Kennung des Autoinjektors als Advertising-Paket via Bluetooth-Einheit im Broadcast-Modus verbreiten, beispielsweise eine Folge von mehrere Paketen hintereinander. Die Kennung des Autoinjektors ist eindeutig und wird idealerweise nur einmal vergeben. In bevorzugten Ausgestaltungen umfassen die Verabreichungsdaten auch einen absoluten oder relativen Zeit- und/oder Datumsstempel. Weiter können die Verabreichungsdaten Temperaturen, Sensordaten, Zeitmessungen, Zustände oder Aktivitäten enthalten.

Das Mobilgerät kann die Übertragung in Form des Advertising-Pakets über die eigene Bluetooth-Einheit empfangen. Auf dem Mobilgerät ist zum Beispiel eine entsprechende App installiert, welche die Daten entgegennimmt. Die App ist jedoch nicht im Besitz eines Schlüssel, welche die Entschlüsselung der verschlüsselten Daten ermöglichen würde. Hingegen erkennt sie die Kennung des Autoinjektors, und kann, wenn der Autoinjektor mehrere Pakete sendet, diese korrekt einordnen. Das mindestens eine empfangene Advertising-Paket kann die App sodann über die Netzwerkeinheit des Mobilgeräts an einen der App bekannten Computer oder Server weiterleiten.

Der entfernte Computer oder Server Computer, nachfolgend Server genannt, kann die vom Mobilgerät weitergeleitete Übermittlung über seine Netzwerkanbindung entgegennehmen. Auf dem Server kann die Gerätekennung gelesen werden. In einer Ausgestaltung ist auf dem Server eine Datenbank abgelegt, in welcher die Kennungen der Geräte hinterlegt sind. Weiter enthält diese Datenbank zu jeder Kennung jeweils einen passenden Schlüssel, mit welchem die verschlüsselten Daten auf dem Server entschlüsselt werden können. In einer alternativen Ausgestaltung kann der Server Zugriff auf einen weiteren Server haben, auf welchem die Datenbank gespeichert ist.

Die entschlüsselten Daten können sodann dazu verwendet werden, um die Verabreichungsdaten zu analysieren, oder zum Beispiel um zu prüfen, wann der Autoinjektor benutzt wurde oder ob eine Lagertemperatur überschritten wurde.

In einer Ausgestaltung der Erfindung bilden der Schlüssel im Verabreichungsgerät zum Verschlüsseln der Daten und der Schlüssel auf dem entfernten Computer oder Server ein asymmetrisches Schlüsselpaar. Dies entspricht den gängigen asymmetrischen Schlüsselpaaren aus öffentlichem und geheimem Schlüssel, wie sie dem Fachmann aus dem Internet bekannt sind. Das hat den Vorteil, dass Daten die mit dem Schlüssel auf dem Verabreichungsgerät verschlüsselt wurden, nur mit dem auf dem Server befindlichen Schlüssel entschlüsselt werden können. Auch wenn ein potentieller Angreifer einen Schlüssel aus dem Verabreichungsgerät extrahieren könnte, so kann er doch nicht auf bereits verschlüsselte Daten zugreifen. Alternativ kann das Schlüsselpaar auch symmetrisch sein, was unter Inkaufnahme des genannten Nachteils ebenfalls funktioniert.

Da das Mobilgerät keinen der Schlüssel hinterlegt, hat es auch keinen Zugriff auf die eigentlichen Verabreichungsdaten. Falls das gewünscht wäre, könnten entschlüsselte Daten, die einem Patienten oder Patientin zur Verfügung gestellten werden sollen, vom Server aufbereitet werden und an die App zurück gesendet werden. Dabei können die Daten zuvor auf dem Server ausgewertet und aufbereitet werden, so dass sie auf der App einfach verständlich dargestellt werden können.

In einer vorteilhaften Ausgestaltung findet zwischen dem Verabreichungsgerät und dem Mobilgerät vor, während und nach einer Datenübertragung kein Bluetooth-Pairing statt. In einer weiteren Fortgestaltung ist es wünschenswert, dass die Bluetooth-Einheit des Verabreichungsgerätes gar nicht im Stande ist ein Pairing durchzuführen, sondern elektronisch auf das nötigste reduziert ist, nämlich die Fähigkeit, Advertising-Pakete zu senden, resp. zu empfangen. In einer noch weiteren ebenfalls vorteilhaften Fortgestaltung wird auch die Empfangsmöglichkeit weggelassen. Diese Reduktion auf das Wesentliche spart Bauteile und reduziert potentielle Fehlerquellen.

In einer weiteren erfindungsgemässen Ausführungsform ist das erfindungsgemässe Verabreichungsgerät modular aufgebaut. Zum Beispiel kann das Verabreichungsgerät aus zwei Modulen ausgebildet sein, welche lösbar miteinander verbunden werden können. So kann ein Modul ein Injektions- oder Infusionsmodul sein und das andere eine Kommunikationsmodul sein. Dabei kann das Kommunikationsmodul insbesondere elektronische Sensorik, eine Steuerung, mindestens einen Prozessor, mindestens ein Speicherbauteil und eine Bluetooth-Einheit enthalten. Das Injektions- oder Infusionsmodul könnte als klassischer Injektions-Pen, als Autoinjektor oder als Patchgerät ohne jegliche Elektronik ausgebildet sein. Das würde zum Beispiel erlauben, das Injektions- oder Infusionsmodul als Einwegmodul zu gestalten und das Kommunikationsmodul als Mehrwegmodul zu wiederholter Verwendung. Die beschriebenen Module könnten jeweils als Komponenten in ein Verabreichungsgerät integriert sein, oder als Zusatzmodul an ein Verabreichungsgerät koppelbar und von diesem wieder lösbar sein.

In einer weiteren erfindungsgemässen Ausführungsform sendet das Mobilgerät nach Erhalt eines Advertising-Pakets von einem Verabreichungsgerät ebenfalls ein Advertising-Paket als Empfangsbestätigung via Bluetooth-Einheit. In einer vorteilhaften Ausführung wird diese Empfangsbestätigung erst dann gesendet, wenn das Mobilgerät seinerseits eine Rückmeldung vom Server bekommen hat, dass das ursprünglich vom Verabreichungsgerät stammende Advertisement-Paket beim Server angekommen ist. In der Empfangsbestätigung an das Verabreichungsgerät wird der Erhalt des Pakets bestätigt. Die Bestätigung enthält im Minimum die Kennung des Verabreichungsgeräts, welches das zu bestätigende Paket gesendet hatte. Typischerweise ist eine eindeutige Kennung des Mobilgerätes angefügt. Im Rahmen der Paketlänge können auch weitere Parameter wie eine Paketkennnummer übertragen werden. Das Verabreichungsgerät kann die Empfangsbestätigung empfangen und auswerten.

Das Mobilgerät kann Advertising-Pakete von mehreren Verabreichungsgeräten empfangen und diese auch bestätigen. Erfindungsgemäss kann auch ein Verabreichungsgerät Advertising-Pakete empfangen, sowohl von einem Mobilgerät, als auch von einem zweiten Verabreichungsgerät.

Sind nun mehrere Verabreichungsgeräte nahe beieinander und können Advertising-Pakete untereinander und Empfangsbestätigungen von mindestens einem Mobilgerät empfangen, so kann es vorteilhaft sein, wenn die Verabreichungsgeräte die im Advertising-Paket enthaltenen oder beim Empfang daraus gewonnenen Zusatzinformationen nutzen.

So kann zum Beispiel ein Verabreichungsgerät, welches solche Zusatzinformationen empfängt, bei der nächsten Übertragung von Verabreichungsdaten, die Verabreichungsdaten mit der Information ergänzen (und verschlüsseln), dass weitere Verabreichungsgeräte in der Nähe entdeckt wurden. Allenfalls kann die ergänzte Information die Kennung des oder der weiteren Verabreichungsgeräte umfassen.

Nähe, resp. das Erkennen der Anwesenheit (also die Präsenz) kann dabei qualitativ oder quantitativ gemeint sein. Qualitativ bedeutet dabei, dass ein Mobilgerät ein Signal von einem Verabreichungsgerät empfängt. Quantitativ bedeutet dabei, dass das Mobilgerät auch ein Mass für die Nähe abschätzen kann. Dies kann zum Beispiel über die RSSI-Funktion geschehen, wobei RSSI Received Signal Strength Indication bedeutet (siehe dazu die Bluetooth Core Spezifikation unter https://www.bluetooth.com/de/specifications/specs/core-specification). Die Version 5.2 vom 31. Dezember 2019 der Bluetooth Core Spezifikation ist hiermit durch Verweis vollständig in das vorliegende Dokument aufgenommen.

Wenn der Server solche Information dann empfängt, kann dieser die Präsenz von zusätzlichen Auswertungen nutzen. Wenn der Server auch die anderen Verabreichungsgeräte kennt (aus der Datenbank), so kann in einer Ausgestaltung die Übertragungszuverlässigkeit der Verabreichungsgeräte erkannt werden. Alternativ oder ergänzend kann auch überprüft werden, ob alle der erkannten Verabreichungsgeräte, insbesondere die darin jeweils enthaltenen Medikamente miteinander verträglich sind. Sind sie es nicht, kann vom Server eine Warnung an die App gesendet werden. In einer weiteren Alternative können vom Server auch Empfehlungen an die App gesendet werden, in welchen zum Beispiel eine bestimmte Reihenfolge der Verabreichung der verschiedenen Medikamente empfohlen wird.

In einer weiteren Ausgestaltung der Erfindung können mehrere Mobilgeräte gleichzeitig Informationen von einem Verabreichungsgerät empfangen. Bei dieser Ausgestaltung ist es besonders vorteilhaft, wenn die Bluetooth-Einheiten der Mobilgeräte die RSSI Funktion (Received Signal Strength Indication) implementiert haben (siehe dazu die Bluetooth Core Spezifikation, Verweis oben erwähnt). Damit ist es dem Mobilgerät möglich, die Empfangsdistanz, also die Distanz zwischen Sender und Empfänger zum Zeitpunkt der Datenübertragung, zwischen dem Verabreichungsgerät und sich selber abzuschätzen. Die so aus der RSSI extrahierte Information kann das Mobilgerät in dieser Ausgestaltung dem weiterzuleitenden Advertisement-Paket beifügen. Auf dem Server können entsprechend identische Advertisement-Pakete, welche über verschiedene Mobilgeräte an den Server weitergeleitet wurden, empfangen werden. Auf dem Server kann dann zum Beispiel entschieden werden, welches der identischen Advertisement-Paket weiterverarbeitet werden soll. Dabei kann die Information, welche aus der RSSI Funktion extrahiert wurde, helfen. Zum Beispiel kann auf dem Server entschieden werden, dass dasjenige Advertisement-Paket aus einer Menge von identischen Advertisement-Paket behalten wird, bei welchem die RSSI-Funktion die kleinste Empfangsdistanz zwischen Mobilgerät und Verabreichungsgerät indiziert. Die restlichen Advertisement-Pakete aus der Menge von identischen Advertisement-Paketen können sodann zum Beispiel verworfen werden. Alternativ können alle Pakete zusammen auch für weitere Auswertungen verwendet werden. Zum Beispiel kann untersucht werden, wie viele Mobilgeräte im Sendebereich eines einzelnen Verabreichungsgeräts vorhanden sind, die die Advertisement-Pakete empfangen und weiterleiten. Rein theoretisch, aber vorteilhaft könnten damit örtliche Häufungen von Patienten und Patienten, die dieselbe Erkrankung behandeln, erkannt werden.

### FIGUREN

In Zusammenhang mit den angehängten Figuren werden nachfolgend bevorzugte Ausführungsformen der Erfindung beschrieben. Diese sollen grundsätzliche Möglichkeiten der Erfindung aufzeigen und keinesfalls einschränkend ausgelegt werden.
- Fig. 1: zeigt ein erstes erfindungsgemässes System bestehend aus einem Patch Injektor 1, einem Smart Phone 13 und einem Server 14.
- Fig. 2: zeigt ein zweites erfindungsgemässes System bestehend aus einem Autoinjektor 11 mit eingebautem Elektronikmodul 11a, einem Smart Phone 13 und einem Server.
- Fig. 3: zeigt ein drittes erfindungsgemässes System bestehend aus einem modularen Injektionsgerät (ein Autoinjektor 11 mit einem Add-on 12), einem Smart Phone und einem Server.
- Fig. 4: zeigt das Flussdiagramm eines erfindungsgemässen Verfahrens zum Übertragen von Verabreichungsdaten zu Dosisverabreichungen aus einem Reservoir eines Verabreichungsgeräts.
- Fig. 5: zeigt ein viertes erfindungsgemässes System, bei welchem mehrere Verabreichungsgeräte über je ein Mobilgerät mit einem Server kommunizieren.
- Fig. 6: zeigt das Flussdiagramm eines erfindungsgemäss erweiterten Verfahrens aus Fig. 4 mit Darstellung der Kommunikation mit zwei verschiedenen Mobilgeräten.

### FIGURENBESCHREIBUNG

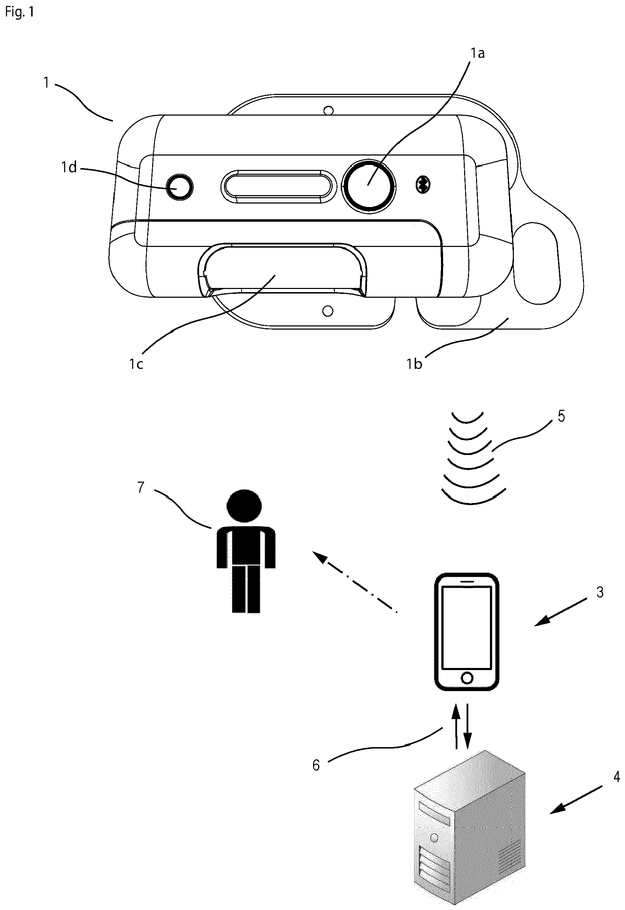

Figur 1 zeigt ein erstes erfindungsgemässes System. Beim erfindungsgemässen Verabreichungsgerät handelt es sich um einen elektronisch betriebenen Patch Injektor 1. Patch Injektoren sind Injektionsgeräte, die direkt auf die Haut des Patienten oder der Patientin mittels Pflaster aufgeklebt werden. Nach dem Aufkleben kann der Injektor 1 durch das Drücken des Startknopfes 1a kann die Injektion des fluiden Medikaments (nicht gezeigt) gestartet werden. Hierbei zeigt zum Beispiel die Status LED 1d den aktuellen Status des Injektor 1 durch einen Farbcode an. Weiter kann der Fortschritt der Injektion auch durch das Sichtfenster 1c, durch welches das Medikamentenreservoir (nicht gezeigt) sichtbar ist, beobachtet werden. Der Injektor umfasst weiter Sensoren (nicht gezeigt), welche die verschiedenen Zustände des Injektors erkennen können. Im Pflaster (nicht sichtbar in Figur 1) des Injektor kann zum Beispiel ein kapazitiver Sensor angeordnet, mit welchem erkannt wird, ob der Injektor auf der Haut klebt. Ein weiter Sensor erkennt das Drücken auf Startknopf 1a und ein noch weiterer Sensor erkennt das Ende einer Injektion (zum Beispiel wenn eine Kolbenstange in einer Spritze/Reservoir eine bestimmte Position erreicht). Wie erwähnt handelt es sich beim Verabreichungsgerät 1 um einen elektronisch betriebenen Injektor. Nebst elektronischer Steuerung und diversen Sensoren enthält Injektor 1 auch einen Speicher, in welchem die Verabreichungsdaten gespeichert werden können, eine eindeutige Kennung des Injektors und einen Schlüssel zum Verschlüsseln der Verabreichungsdaten. Auch eine Bluetooth-Einheit (nicht gezeigt) ist in den Injektor 1 integriert und mit der Steuerung verbunden. Die Steuerung kann nun, sobald aktiviert (zum Beispiel durch das Abziehen der Schutzfolie 1b), laufend die Sensoren abfragen und allfällige Zustandsänderungen als verschlüsselte Daten zusammen mit der eindeutigen Kennung via die Bluetooth-Einheit als Advertising-Pakete über einen Broadcast-Kanal 5 versenden. Kommt keine Bestätigung vom Mobilgerät oder Smart Phone 3, so kann ein Paket wiederholt versandt werden. Das Mobilgerät 3 kann die Advertising-Pakete dann jeweils über die Netzwerkverbindung 6 an den Server 4 senden.

Der Server 4 kann wie beschrieben aufgrund der in einer Datenbank (nicht gezeigt) vorhandenen Informationen zu Kennung und passendem Schlüssel die Daten entschlüsseln und auswerten. Alternativ kann die zur Steuerung und Kommunikation notwendige Elektronik in Form eines Elektronik-Moduls 11a in den Patch Injektor integriert sein.

Eine dem Fachmann naheliegende Ausführungsform (nicht gezeigt) des Patch Injektors 1 ist ein modularer Aufbau des Verabreichungsgerätes 1 mit einer Reservoir-Einheit mit dem Reservoir zum Einweggebrauch und eine wiederverwendbare Antriebseinheit mit dem Antrieb, wobei der Speicher mit dem Schlüssel in der Reservoir-Einheit vorgesehen ist.

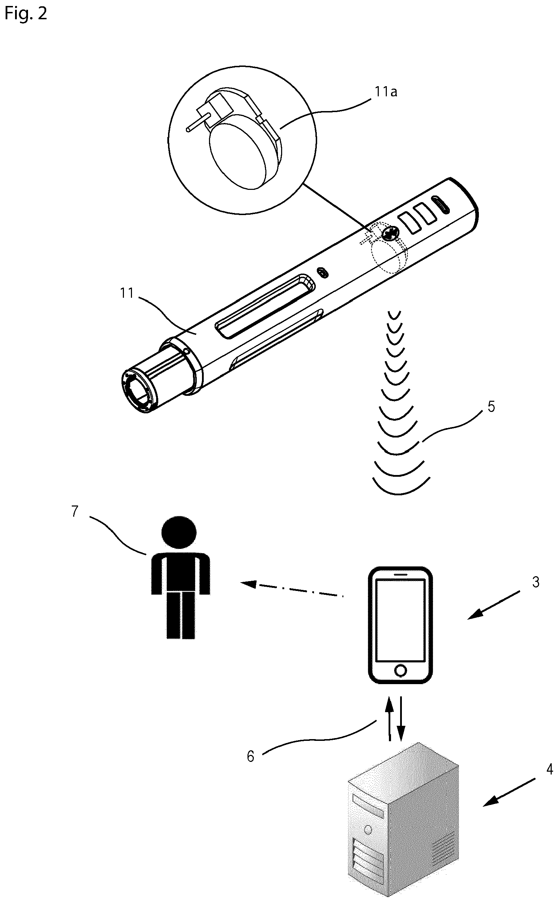

Fig.2 zeigt ein Verabreichungsgerät in Form eines Autoinjektors 11 mit integriertem Elektronikmodul 11a.

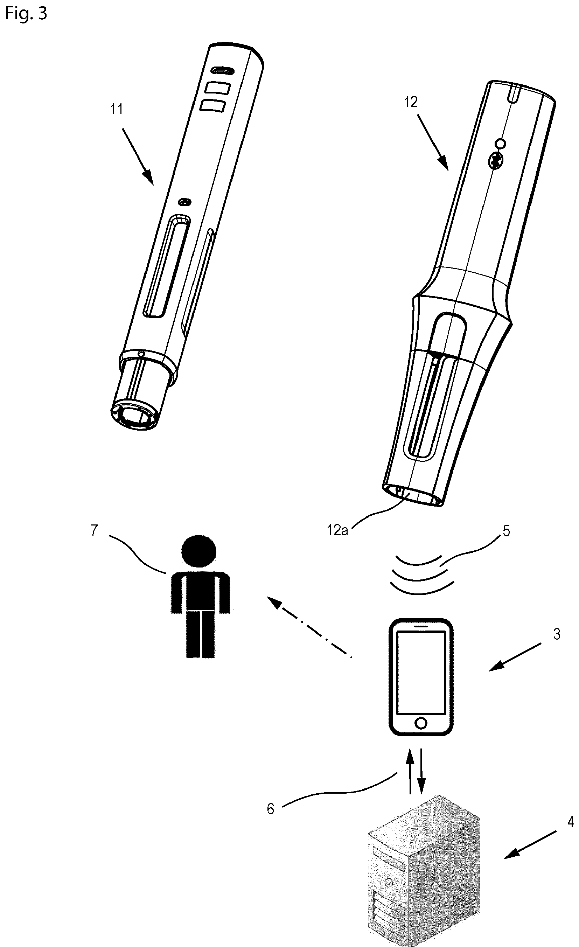

Figur 3 zeigt ein zweites Erfindungsgemässes System. Bei dieser Ausführungsform ist das System modular aufgebaut und besteht aus einem Autoinjektor 11 ohne Elektronikmodul, und einem Zusatzmodul 12. Der Autoinjektor 11 kann dabei mit seinem hinteren Ende in die Öffnung 12a des Add-ons 12 hingeschoben werden. Die beiden Teile können miteinander verschnappt (nicht gezeigt) werden, so dass eine Einheit entsteht. Das Add-on 12 umfasst die erfindungsgemässen Elemente des Elektronikmoduls 11a mit Bluetooth-Einheit, und kann den Schlüssel aus einem NFC Speicher des Autoinjektors zur eindeutigen Identifikation des Reservoirs auslesen. Die Einheit aus Add-on 12 und Autoinjektor 11 funktioniert bezüglich der Erfindung gleich wie das System aus Figur 1. Verschlüsselte Daten werden zusammen mit der Kennung in Advertising-Paketen über einen Bluetooth Broadcast-Kanal 5 drahtlos verbreitet. Das Mobilgerät, hier in Figur 3 auch ein Smart Phone 3, leitet die Pakete über das Netzwerk 6 weiter an den Server 4.

Von Server 4 können ausgewertete Daten allenfalls via Netzwerk 6 zurück an das Smart Phone 3 gesendet werden, welche dann von der Person 7 auf dem Smart Phone angeschaut werden können.

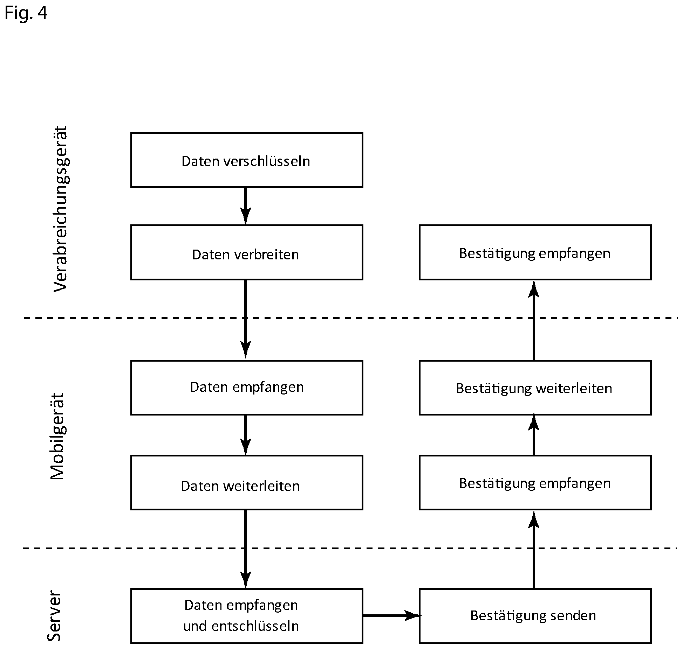

Figur 4 zeigt ein Flussdiagram für ein erfindungsgemässes Verfahren zum Übertragen von Verabreichungsdaten zu Dosisverabreichungen aus einem Reservoir eines Verabreichungsgeräts 1 mit den folgenden Schritten:
- Verschlüsseln der Verabreichungsdaten mit Hilfe eines Schlüssels auf einem Prozessor des Verabreichungsgeräts 1. Verabreichungsdaten sind insbesondere Angaben zur Qualität, zum Erfolg, zu Medikamentenvolumen, zum Zeitpunkt oder Dauer einer Injektion oder von Teilschritten eines zum Zweck einer Verabreichung durchgeführten Bedien-Ablaufs am Verabreichungsgerät 1. Der auf dem Verabreichungsgerät 1 gespeicherte Schlüssel kann zum Beispiel ein asymmetrisches Schlüsselpaar bilden mit einem zweiten Schlüssel, der in einer Datenbank auf dem Server-Computer 4 gespeichert ist. Die Verteilung der Schlüssel ist so gewählt, dass die auf dem Verabreichungsgerät 1 verschlüsselten Daten nur mit dem passenden Schlüssel des Server-Computers 4 entschlüsselbar sind. Andere Geräte, die an der Kommunikation oder Speicherung der Verabreichungsdaten beteiligt sind, insbesondere das Mobilgerät 3, verfügen typischerweise nicht über den passenden Schlüssel.
- Drahtloses Verbreiten der verschlüsselten Verabreichungsdaten zusammen mit einer eindeutigen Kennung des Verabreichungsgeräts 1 in einem Broadcast-Modus als verbreitete Daten über eine Bluetooth-Einheit des Verabreichungsgeräts 1 und einen Bluetooth Broadcast-Kanal 5. Das sind typischerweise BLE Advertisement Frames mit Datenpaketen ohne Empfängerangabe, sodass eine vorhergehende Verbindungsaufnahme (Pairing) nicht notwendig ist.
- Empfangen der verbreiteten Daten, der eindeutigen Kennung und allfälliger weiterer Bestandteile eines Datenpaketes über eine Bluetooth-Einheit auf einem Mobilgerät 3. Weil die Daten ohne vorherige Verbindungsaufnahme übertragen werden können, gehört dazu auch eine laufende Verfolgung des drahtlosen Datenverkehrs in der Umgebung des Mobilgerätes 3, sowie eine Analyse desselben, sodass Verabreichungsdaten eines Verabreichungsgerätes 1 als solche erkannt werden. Ebenso ist es möglich, den Benutzer 7 hier in den Ablauf mit einzubeziehen und zum Beispiel eine manuelle Bestätigung anzufordern. Entschlüsseln von Daten ist dazu nicht notwendig.
- Weiterleiten der verbreiteten Daten, insbesondere der verschlüsselten Verabreichungsdaten, vom Mobilgerät 3 über eine Netzwerkschnittstelle des Mobilgerätes 3 an einen entfernten Server-Computer 4. Der Server-Computer 4 kann auch auf einer Mehrzahl von Prozessoren oder Computern realisiert sein, die zum Zwecke der Verarbeitung von Verabreichungsdaten zusammenarbeiten. Das Mobilgerät 3 kann die übermittelten Verabreichungsdaten typischerweise selber nicht entschlüsseln, weil der dazu nötige Schlüssel erfindungsgemäss nur auf dem Server-Computer 4 zur Verfügung steht.
- Empfangen und Entschlüsseln der in den verbreiteten Daten verschlüsselten Verabreichungsdaten durch den Server-Computer 4 mit Hilfe eines auf dem Server-Computer 4 gespeicherten passenden Schlüssels, welcher anhand der eindeutigen Kennung identifiziert wird. Die für den Betrieb notwendigen Schlüssel können zum Beispiel bei der Installation oder Inbetriebnahme des Systems auf dem Server-Computer 4 bereitgestellt werden
- Senden, durch den Server-Computer 4, einer Empfangs-Bestätigung an das Mobilgerät 3. Die Empfangs-Bestätigung umfasst im Minimum eine eindeutige Kennung des Verabreichungsgerätes 1, kann aber auch weitere Daten enthalten, wie die Kennung des Mobilgerätes 3, Elemente aus dem Bluetooth Kommunikationsprotokoll oder Verabreichungsdaten.
- Empfangen und Weiterleiten der Empfangs-Bestätigung durch das Mobilgerät 3 an das Verabreichungsgerät 1 über den Bluetooth Broadcast-Kanal 5. Dazu ist wiederum keine Verbindungsaufnahme (Pairing) notwendig. Das Mobilgerät 1 kann die Empfangs-Bestätigung zusätzlich auch auf einem Bildschirm anzeigen oder auf andere Weise dem Benutzer 7 zur Kenntnis bringen. Auch hier ist es möglich, den Benutzer 7 in den Ablauf mit einzubeziehen und zum Beispiel eine manuelle Bestätigung anzufordern, entweder bei jeder Datenübertragung, oder auch selektiv je nach Beurteilung der Übertragungsbedingungen.
- Empfangen der Empfangs-Bestätigung durch das Verabreichungsgerät 1.

Die Rückmeldung einer Bestätigung vom Server 4 an das Verabreichungsgerät 1 (rechte Seite im Flussdiagram von Fig. 4) ist dabei nicht zwingend notwendig, für die Absicherung der Übertragung jedoch vorteilhaft und bevorzugt. In gleicher Weise kann auch die manuelle Bestätigung von Datenpaketen und Steuerung der Kommunikation durch den Benutzer 7 wegfallen oder an verschiedenen Stellen des beschriebenen Ablaufs erfolgen. In oben angegebenem Ablauf sind zwei Positionen beispielhaft erwähnt, die sich für die Absicherung einer Datenübertragung besonders eignen.

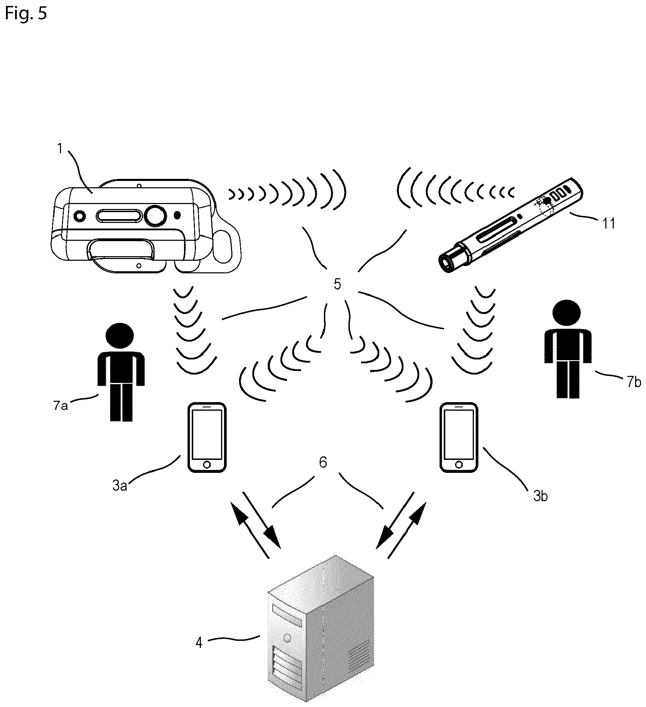

Figur 5 zeigt ein System nach der vorliegenden Erfindung, bei welchem mehrere Verabreichungsgeräte 1, 11 über je mindestens ein Mobilgerät 3a, 3b mit dem Server-Computer 4 kommunizieren. Wiederum ist für die Kommunikation keine explizite Zuordnung (Pairing) zwischen Mobilgerät 3a, 3b und Verabreichungsgerät 1, 11 notwendig, was die Komplexität der Bedienung deutlich reduziert. Zur Absicherung der Übertragung von Verabreichungsdaten an den Server 4, oder auch zur Darstellung gerätespezifischer Information auf dem Mobilgerät 3a, 3b ist eine solche Zuordnung sehr nützlich. Eine Erweiterung des Ablaufs von Fig. 4 erlaubt nun, dass die Verabreichungsgeräte 1, 11 innerhalb eines Bluetooth-Kommunikationsbereichs eine solche Zuordnung unterstützen. In Fig. 5 ist auch erkennbar, dass Verabreichungsgeräte 1, 11, die in genügend kleinem Abstand voneinander benutzt werden, auch untereinander kommunizieren können. So sind Verabreichungsdaten und sämtliche vom Verabreichungsgerät 1 oder Mobilgerät 3a im Broadcast Modus gesendete Information auch vom Verabreichungsgerät 11 empfangbar und kann zur Absicherung des Systems, zur Unterstützung der Zuordnung zwischen Mobilgerät 3a, 3b und Verabreichungsgerät 1, 11, aber auch zur Realisierung weiterer Funktionen wie zum Beispiel einer grafischen Darstellung der Netzwerktopologie benutzt werden. Analog können beide Verabreichungsgeräte 1, 11 auch Bestätigungen von beiden Mobilgeräten 3a, 3b empfangen und für die Realisierung der erwähnten Funktionen benutzen. Insbesondere ist es möglich, dass jedes Verabreichungsgerät 1, 11 für jedes über einen Bluetooth Broadcast Kanal 5 empfangene Datenpaket eine Abschätzung macht, wie gross für diese Daten die Empfangsdistanz ist - also die Distanz zwischen Sender und Empfänger - sei es ein Mobilgerät 3a, 3b oder ein weiteres Verabreichungsgerät 1, 11. Die Auswertung dieser Information ist ein weiterer wichtiger Aspekt der vorliegenden Erfindung. Im einfachsten Fall kann das Verabreichungsgerät 1, 11 eine nicht weiter spezifische Warnung ausgeben, dass sich mehrere Verabreichungsgeräte 1, 11 oder Mobilgeräte 3a, 3b in seiner Nähe befinden.

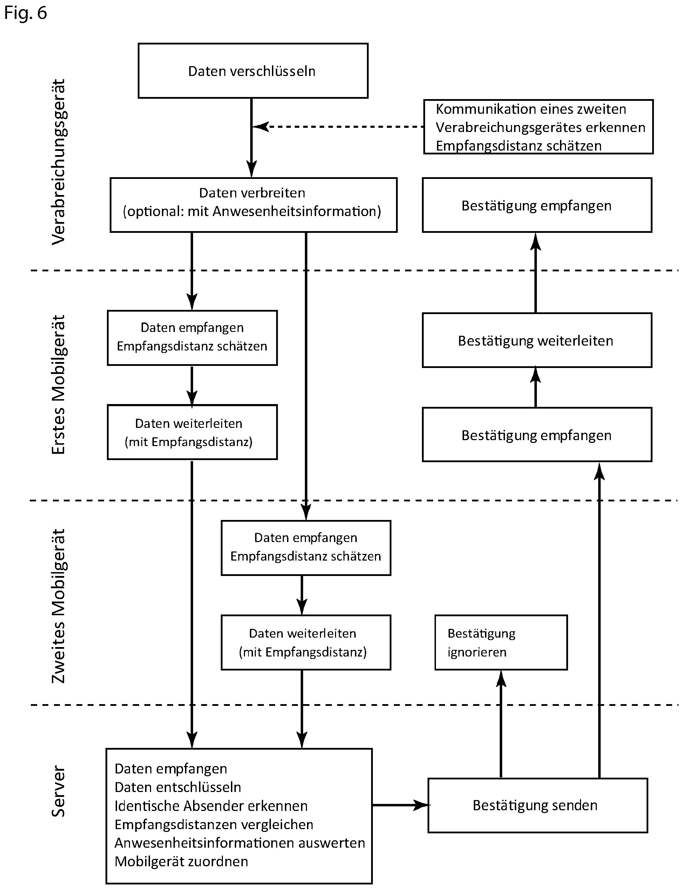

Figur 6 zeigt ein erweitertes Flussdiagramm, welches zusätzlich zu dem in Fig. 4. beschriebenen Ablauf die folgenden Schritte enthält:
- Empfangen der verbreiteten Daten über eine Bluetooth-Einheit auf einem ersten Mobilgerät 3a, Abschätzen einer ersten Empfangsdistanz zwischen dem ersten Mobilgerät 3a und dem ersten Verabreichungsgerät 1 basierend auf dem Empfang der verbreiteten Daten, und Weiterleiten der ersten Empfangsdistanz an den Server-Computer 4. Die Abschätzung der Empfangsdistanz kann mittels Auswertung der gemäss Bluetooth-Standard mitgesendeten Received Signal Strength Indication (RSSI) erfolgen, oder durch Auswertung einer anderen geeigneten Information, wie zum Beispiel Bit Error Rate (BER) oder Quality of Service (QoS). Ein Mass für die Empfangsdistanz wird dabei den Verabreichungsdaten und der Kennung des Verabreichungsgerätes 1 angehängt oder damit kombiniert, sodass alle drei Informationen dem Mobilgerät 3a und dem Server-Computer 4 zur Verfügung stehen.
- Empfangen der verbreiteten Daten über eine Bluetooth-Einheit auf einem zweiten Mobilgerät 3b, Abschätzen einer zweiten Empfangsdistanz zwischen dem zweiten Mobilgerät 3b und dem Verabreichungsgerät 1 basierend auf dem Empfang der verbreiteten Daten, und Weiterleiten der zweiten Empfangsdistanz an den Server-Computer 4. Wiederum kann diese Abschätzung auf der RSSI von Bluetooth beruhen oder auf einer anderen geeigneten Information. Falls sich wie abgebildet alle Geräte in Reichweite einer Bluetooth-Verbindung befinden, so analog auch die Verabreichungsdaten des zweiten Verabreichungsgerätes 11 von beiden Mobilgeräten 3a, 3b empfangen und zusammen mit einem Mass für die geschätzte Empfangsdistanz zwischen Mobilgerät und Verabreichungsgerät an den Server weitergeleitet. Nur schon die Information, dass sich zwei verschiedene Verabreichungsgeräte in der Nähe eines Mobilgerätes befinden, ist für die Zuordnung von Bedeutung und kann zur Absicherung der Kommunikation verwendet werden.
- Empfangen und als identisch Erkennen der vom ersten und zweiten Mobilgerät 3a, 3b weitergeleiteten Verabreichungsdaten auf dem Server-Computer 4. Der Server-Computer 4 kann dazu den Absender der Verabreichungsdaten an der mitgeschickten Identifikation oder an einem beliebigen anderen Teil der Daten erkennen.
- Zuordnen, auf dem Server-Computer 4, der von dem ersten oder zweiten oder beiden Mobilgeräten 3a, 3b empfangenen Verabreichungsdaten des Verabreichungsgerätes 1 an erste oder das zweite Mobilgerät 3a, 3b durch Vergleich der ersten und der zweiten Empfangsdistanz. Nachdem die Zuordnung erfolgt ist, wird die Bestätigung nur noch an genau ein Mobilgerät 3a oder 3b übermittelt; im Beispiel von Figur 5 ist dies das erste Mobilgerät 3a. Analog erfolgt die Bestätigung an das zweite Mobilgerät 3b falls die an den Server 4 übermittelten Empfangsdistanzen ergeben, dass sich das zweite Mobilgerät 3b näher am ersten Verabreichungsgerät 1 befindet. Wiederum analog kann eine Zuordnung der Verabreichungsdaten von Verabreichungsgerät 11 an das nächstliegende Mobilgerät 3b erfolgen.

Ebenfalls in Fig. 6 ist dargestellt, dass ein Verabreichungsgerät 1 auch Bluetooth Advertising-Kanäle nach Datenpaketen anderer Kommunikationspartner in der Umgebung wahrnehmen, empfangen und analysieren kann. In gleicher Weise wie Bestätigungen von einem Mobilgerät 3 empfangen werden, kann das auch für Bestätigungen, Datenpakete oder sonstige Signale geschehen, die nicht für das betreffende Verabreichungsgerät 1 bestimmt sind, sondern zum Beispiel für ein zweites Verabreichungsgerät 11. Das Verabreichungsgerät 1 kann vorteilhaft eine solche Kommunikation als solche erkennen und daraus die Anwesenheit eines zweiten Verabreichungsgerätes 11 innerhalb der Bluetooth-Reichweite ableiten. Bevorzugt wird eine solche Anwesenheit auch quantifiziert, zum Beispiel durch Schätzen der Empfangsdistanz. In einer weiteren Ausführungsform fügt das Verabreichungsgerät 1 die Anwesenheitsinformation seinen Verabreichungsdaten beim Senden über den Bluetooth Kanal 5 hinzu. Das Mobilgerät 3 leitet diese an den Server 4 weiter, und der Server 4 wertet die Anwesenheitsinformation aus. In gleicher Art wie der Server 4 die Empfangsdistanzen der verschiedenen Geräte in der Reichweite der Bluetooth - Technologie zur Zuordnung von Mobilgeräten 3 und Verabreichungsdaten benützt, kann der Server 4 allfällige quantifizierte oder nicht quantifizierte Anwesenheitsinformationen zur Bildung oder Überprüfung von Zuordnungen verwenden und damit die Sicherheit der Kommunikation erhöhen.

### BEZUGSZEICHENLISTE

- 1: Verabreichungsgerät, Patch Injektor (Pflasterartiges Injektionsgerät)
- 1a: Auslöseknopf
- 1b: Schutzfolie Pflaster
- 1c: Sichtfenster Reservoir
- 1d: Status LED
- 3, 3a, 3b: Mobilgerät, Smart Phone
- 4: Server, Server-Computer oder Cloud
- 5: Bluetooth Broadcast-Kanal, uni- oder bidirektional
- 6: Netzwerk und/oder Mobilfunk/Mobiltelefonverbindung
- 7, 7a, 7b: Nutzende Person oder Patient
- 11: Verabreichungsgerät, Autoinjektor
- 11a: Elektronikmodul
- 12: Add-on
- 12a: Öffnung

## Patentansprüche

1. Verfahren zum Übertragen von Verabreichungsdaten zu einer Dosisverabreichung aus einem Reservoir eines Verabreichungsgeräts 1, wobei das Verfahren folgende Schritte umfasst:
- Verschlüsseln der Verabreichungsdaten mit Hilfe eines Schlüssels auf einem Prozessor des Verabreichungsgeräts 1,
- drahtloses Verbreiten der verschlüsselten Verabreichungsdaten zusammen mit einer eindeutigen Kennung des Verabreichungsgeräts 1 in einem Broadcast-Modus als verbreitete Daten über eine Bluetooth-Einheit des Verabreichungsgeräts 1 und einen Bluetooth Broadcast-Kanal 5,
- Empfangen und Weiterleiten der verbreiteten Daten und der eindeutigen Kennung des Verabreichungsgerätes 1 durch ein Mobilgerät 3 über eine Netzwerkschnittstelle des Mobilgerätes 3 an einen entfernten Server-Computer 4,
- Empfangen und Entschlüsseln der in den verbreiteten Daten verschlüsselten Verabreichungsdaten durch den Server-Computer 4 mit Hilfe eines auf dem Server-Computer 4 gespeicherten passenden Schlüssels, welcher anhand der eindeutigen Kennung identifiziert wird,
- Senden, durch den Server-Computer, einer Empfangs-Bestätigung an das Mobilgerät unter Angabe der eindeutigen Kennung des Verabreichungsgerätes.

2. Verfahren nach Anspruch 1, wobei das Verfahren zusätzlich die folgenden Schritte umfasst:
- Empfangen und Weiterleiten der Empfangs-Bestätigung durch das Mobilgerät 3 an das Verabreichungsgerät 1 über einen Bluetooth Broadcast-Kanal 5, und
- Empfangen der Empfangs-Bestätigung durch das Verabreichungsgerät 1.

3. Verfahren nach Anspruch 1 oder 2, wobei eine benutzende Person 7 die erfolgte Dosisverabreichung auf dem Mobilgerät 3 manuell bestätigt.

4. Verfahren nach Anspruch 1 oder 2, wobei der Schlüssel im Speicher des Verabreichungsgeräts 1 und der passende Schlüssel im Speicher oder einer Datenbank des Server-Computers 4 ein asymmetrisches Schlüsselpaar bilden, und Verabreichungsdaten, die auf dem Verabreichungsgerät 1 verschlüsselt wurden, nur mit dem passenden Schlüssel des Server-Computers 4 entschlüsselbar sind.

5. Verfahren nach Anspruch 1 oder 2, wobei das Mobilgerät 3 die verschlüsselten Verabreichungsdaten unverändert weiterleitet, insbesondere nicht entschlüsselt.

6. Verfahren nach Anspruch 1 oder 2, wobei zwischen dem Verabreichungsgerät 1 und dem Mobilgerät 3 vor dem Verbreiten und Empfangen kein Bluetooth-Pairing stattfindet.

7. Verfahren nach einem der Ansprüche 1 bis 6, weiter umfassend folgende Schritte:
- Empfangen der verbreiteten Daten über eine Bluetooth-Einheit auf einem ersten Mobilgerät 3a, Abschätzen einer ersten Empfangsdistanz zwischen dem ersten Mobilgerät 3a und dem Verabreichungsgerät 1 basierend auf dem Empfang der verbreiteten Daten, und Weiterleiten der ersten Empfangsdistanz an den Server-Computer 4,
- Empfangen der verbreiteten Daten von Verabreichungsgerät 1 über eine Bluetooth-Einheit auf einem zweiten Mobilgerät 3b, Abschätzen einer zweiten Empfangsdistanz zwischen dem zweiten Mobilgerät 3b und dem Verabreichungsgerät 1 basierend auf dem Empfang der verbreiteten Daten, und Weiterleiten der zweiten Empfangsdistanz an den Server-Computer 4,
- Empfangen und als identisch Erkennen der von dem ersten oder zweiten oder beiden Mobilgeräten 3a, 3b empfangenen Verabreichungsdaten auf dem Server-Computer 4,
- Zuordnen, auf dem Server-Computer 4, der von dem ersten oder zweiten oder beiden Mobilgeräten 3a, 3b empfangenen Verabreichungsdaten an das erste oder das zweite Mobilgerät 3a, 3b durch Vergleich der ersten und der zweiten Empfangsdistanz.

8. Verfahren nach Anspruch 7, wobei das Abschätzen der ersten oder zweiten Empfangsdistanz mittels Bluetooth Received Signal Strength Indication (RSSI) vorgenommen wird.

9. Verfahren nach einem der Ansprüche 1 bis 8, wobei ein erstes Verabreichungsgerät 1 in einem Bluetooth Broadcast-Kanal 5 eine Kommunikation mit einem zweiten Verabreichungsgerät 11 erkennt und daraus eine Anwesenheit des zweiten Verabreichungsgeräts 11 innerhalb einer Bluetooth-Reichweite ableitet.

10. Verfahren nach Anspruch 9, wobei das erste Verabreichungsgerät 1 über den Bluetooth Broadcast-Kanal 5 Empfangs-Bestätigungen empfängt und auswertet, welche für das zweite Verabreichungsgerät 11 bestimmt sind.

11. Verfahren nach Anspruch 9 oder 10, wobei das erste Verabreichungsgerät 1 den selber gesendeten Verabreichungsdaten eine Anwesenheitsinformation hinzufügt, welche die Anwesenheit des zweiten Verabreichungsgeräts 11 anzeigt.

12. Verfahren nach Anspruch 9, 10 oder 11, wobei die Anwesenheitsinformation eine Abschätzung dafür enthält, wie nahe sich das zweite Verabreichungsgerät 11 oder ein Mobilgerät 3 am ersten Verabreichungsgerät befindet.

13. Verfahren nach Anspruch 12, wobei auf dem Server-Computer 4 Empfangsdistanz-Abschätzungen verschiedener Verabreichungsgeräte 1, 11 zur Überprüfung der Zuordnung der Verabreichungsgeräte 1, 11 zu je einem Mobilgerät 3 verwendet werden.

14. Ein Injektionssystem mit
einem Verabreichungsgerät 1 mit einem Antrieb zur subkutanen Abgabe von mindestens einer Dosis eines fluiden Medikaments aus einem Reservoir,
ein Elektronikmodul 11a mit Sensorik zum Detektieren von Abgabevorgängen, Verarbeitungselektronik zum Auswerten der Daten aus der Sensorik und mit einer drahtlosen Bluetooth-Sendevorrichtung zur drahtlosen Übertragung von Information,
wobei das Injektionssystem über einen Speicher verfügt, in welchem ein Schlüssel ablegbar ist, der das Reservoir eindeutig identifiziert, und welcher zum Verschlüsseln der drahtlos übertragenen Information nutzbar ist, und
wobei die Bluetooth-Sendevorrichtung so gestaltet ist, dass die verschlüsselte Information zusammen mit einer nicht-verschlüsselten, eindeutigen Kennung des Reservoirs wiederholt drahtlos übertragbar ist.

15. Injektionssystem nach Anspruch 14, wobei das Verabreichungsgerät 1 modular aufgebaut ist und eine Reservoir-Einheit mit dem Reservoir zum Einweggebrauch und eine wiederverwendbare Antriebseinheit mit dem Antrieb umfasst, und wobei der Speicher mit dem Schlüssel in der Reservoir-Einheit vorgesehen ist.
